# FASCICULE DE BREVET EUROPEEN

(11) **EP 2 116 247 B1**
(45) Date de publication et mention de la délivrance du brevet: **04.03.2015**
(21) Numéro de dépôt: 09159440.8
(22) Date de dépôt: 05.05.2009
(51) Int. Cl.: A61K 31/517, A61K 31/536, A61K 45/06, A61P 25/28

(54) **Composés anti-amnésiants et compositions pharmaceutiques les comprenant**
Amnäsieverhindernde Verbindungen und sie enthaltende pharmazeutische Zusammensetzungen
Anti-amnesia compounds and pharmaceutical compositions comprising same

(30) Priorité: 06.05.2008 FR 0853003
(43) Date de publication de la demande: 11.11.2009
(73) Titulaire: Biocodex, 94250 Gentilly (FR)
(72) Inventeur: Verleye, Marc, 60190 Remy (FR); Le Guern, Marie-Emmanuelle, 60200 Compiegne (FR); Gillardin, Jean-Marie, 60680 Jonquieres (FR); Hublot, Bernard, 60200 Compiegne (FR)
(74) Mandataire: Vial, Lionel

(56) Documents cités:
- EP-A- 1 745 786
- WO-A-2007/000393
- WO-A-2007/109288
- WO-A-2007/109289
- FR-M- 7 358
- BESNIER N ET AL: "Etifoxine: recent clinical studies" ENCEPHALE, PARIS, FR, no. Suppl. 1, 1 janvier 2008 (2008-01-01), page S9, XP008098900 ISSN: 0013-7006
- MICALLEF J ET AL: "A double blind parallel group placebo controlled comparison of sedative and amnesic effects of etifoxine and lorazepam in healthy subjects" FUNDAMENTAL & CLINICAL PHARMACOLOGY, ELSEVIER, PARIS, FR, vol. 15, no. 3, 1 janvier 2001 (2001-01-01), pages 209-216, XP008098902 ISSN: 0767-3981
- KOISTINAHO M ET AL: "Specific spatial learning deficits become severe with age in beta -amyloid precursor protein transgenic mice that harbor diffuse beta -amyloid deposits but do not form plaques.", PROCEEDINGS OF THE NATIONAL ACADEMY OF SCIENCES OF THE UNITED STATES OF AMERICA 4 DEC 2001 LNKD- PUBMED:11724968, vol. 98, no. 25, 4 December 2001 (2001-12-04), pages 14675-14680, ISSN: 0027-8424
- YASUNO FUMIHIKO ET AL: "Inhibitory effect of hippocampal 5-HT1A receptors on human explicit memory.", THE AMERICAN JOURNAL OF PSYCHIATRY FEB 2003 LNKD- PUBMED:12562581, vol. 160, no. 2, February 2003 (2003-02), pages 334-340, ISSN: 0002-953X
- KENNETT G A ET AL: "Antidepressant-like action of 5-HT1A agonists and conventional antidepressants in an animal model of depression.", EUROPEAN JOURNAL OF PHARMACOLOGY 24 FEB 1987 LNKD- PUBMED:2883013, vol. 134, no. 3, 24 February 1987 (1987-02-24), pages 265-274, ISSN: 0014-2999
- BONTEMPI BRUNO ET AL: "Cognitive enhancing properties and tolerability of cholinergic agents in mice: a comparative study of nicotine, donepezil, and SIB-1553A, a subtype-selective ligand for nicotinic acetylcholine receptors.", NEUROPSYCHOPHARMACOLOGY : OFFICIAL PUBLICATION OF THE AMERICAN COLLEGE OF NEUROPSYCHOPHARMACOLOGY JUL 2003 LNKD- PUBMED:12700710, vol. 28, no. 7, July 2003 (2003-07), pages 1235-1246, ISSN: 0893-133X

## Description

### Domaine de l'invention

La présente invention concerne l'utilisation de nouveaux composés anti-amnésiants dans le cadre de la prévention ou du traitement des troubles de la mémoire, ou troubles mnésiques.

### Arrière-plan de l'invention

L'étiologie des troubles de la mémoire est particulièrement diverse.

Ainsi, parmi les pathologies les plus communes dans lesquelles un trouble mnésique est présent, on peut citer les démences, telle que la maladie d'Alzheimer, les traumatismes cérébraux, les infections cérébrales, telles que les encéphalites ou les méningites, ainsi que les accidents cérébrovasculaires, ischémiques ou hémorragiques. Le trouble mnésique peut être liée à une carence biochimique, comme dans le cas du syndrome de Wernicke-Korsakoff, dû à une carence grave en vitamine B1 du fait d'un alcoolisme chronique. Par ailleurs, le trouble mnésique peut être d'origine iatrogène, il peut notamment être provoqué par certaines substances stupéfiantes et/ou pharmacologiquement actives, comme les sédatifs, les benzodiazépines et substances apparentées utilisées comme hypnotiques, les antinauséeux et antivertigineux, du fait de leur action neuroleptique, les antidépresseurs à action anticholinergique, les antihypertenseurs à action centrale ou les bétabloquants franchissant la barrière hématoencéphalique.

Les troubles mnésiques englobent notamment les différents types d'amnésie.

La plus commune est l'amnésie antérograde, caractérisée par une incapacité à stocker, retenir ou se rappeler de nouvelles connaissances après l'événement déclencheur de l'amnésie. Ce type d'amnésie est notamment observé chez des patients atteints de démence ou de maladie d'Alzheimer.

L'amnésie rétrograde est quant à elle, une perte des souvenirs acquis avant le début des troubles. Elle est souvent observée après un traumatisme cérébral.

Enfin, l'amnésie globale transitoire est une perte temporaire totale de mémoire avec une incapacité à former de nouveaux souvenirs accompagnée d'une perte modérée des souvenirs passés. Cette forme rare, qui régresse spontanément, est observée principalement chez les personnes âgées mais elle peut aussi être causée par des migraines, des accidents vasculaires situés dans le lobe temporal, ou des attaques ischémiques transitoires.

Les traitements possibles des troubles mnésiques dépendent en général de la cause de ces troubles.

Ainsi, dans le cas du syndrome de Korsakoff, des injections de vitamine B1 à haute dose permettent de réduire le trouble mnésique caractéristique de ce syndrome.

Les troubles mnésiques dus à la maladie d'Alzheimer quant à eux, peuvent être partiellement réduits avec du donépézil et des médicaments qui augmentent la fonction cholinergique du cerveau. Deux demandes internationales font également état de la mise au point de peptides ou polypeptides efficaces dans le traitement des troubles mnésiques liée à la maladie d'Alzheimer, soit en agissant sur les effets amnésiques de la protéine β amyloïde (WO/1995/008999), soit en agissant comme agonistes des récepteurs de protéines G (WO/1996/039439).

Différentes études ont également été réalisées pour déterminer les modes de traitement des troubles mnésiques post-traumatiques. Ainsi, un traitement combiné de la physostigmine et de la lécithine peut améliorer les capacités de stockage et de récupération des mots dans la mémoire verbale (Goldberg et al. (1982) J. Clin. Neuropsychol. 4:219-234). Il a également pu être montré que le donépézil a amélioré la mémoire de deux patients amnésiques suite à un traumatisme cérébral (Taverni et al., (1998) Brain Injury 12:77-80).

Enfin, plusieurs études ont porté sur les troubles mnésiques iatrogènes. Ces troubles mnésiques sont ainsi inversés grâce à un traitement combiné à la tacrine et la galantamine et atténués par le piracetam ou la dihydroergocristine (Chopin et Briley, (1992) Psychopharmacology 106:26-30). On observe également un effet positif de l'acéclidine (Mashkovskii et al., (1997) Rev. Bull. Exp. Biol. Med. 123:296-298). Plus généralement, la galantamine peut être utilisée pour tous les troubles mnésiques dus à des substances psychotropes (EP1383507).

Cependant ces différents agents ne sont pas efficaces dans toutes les situations. Ils sont de plus responsables d'un certain nombre d'effets indésirables, en particulier des troubles digestifs.

Un objet de la présente invention est donc de fournir des compositions pharmaceutiques utiles pour prévenir ou traiter les différents types de troubles mnésiques.

L'étifoxine, ou chlorhydrate de 6-chloro-2-éthylamino-4-méthyl-4-phényl-4H-[3,1]benzoxazine appartient à la famille des benzoxazines. Elle favorise la transmission gabaergique en se liant au site du canal chlore couplé au récepteur GABA(A) et est actuellement utilisée comme anxiolytique. Peu de manifestations indésirables consécutives à son utilisation sont répertoriées.

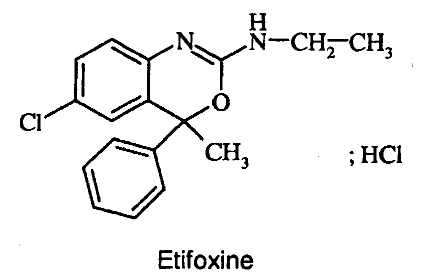

La synthèse de ce composé est notamment décrite dans le brevet français n° 1 571 287. Par ailleurs, plusieurs métabolites actifs de l'étifoxine ont été décrits, tels que la déséthyl-étifoxine ou 2-amino-6-chloro-4-méthyl-4-phényl-4H-[3,1]benzoxazine, la 6-chloro-4-(4-hydroxyphényl)-4-méthyl-3,4-dihydro-1H-quinazolin-2-one ou la 6-chloro-3-éthyl-7-hydroxy-4-méthyl-4-phényl-3,4-dihydro-1 H-quinazolin-2-one.

### Résumé de l'invention

La présente invention découle de la mise en évidence, par les inventeurs, que l'étifoxine possède une action anti-amnésiante chez l'animal dans un modèle de trouble mnésique induit par la scopolamine.

L'invention est définie par les revendications 1 à 14.

La présente invention concerne ainsi l'utilisation d'au moins un composé de formule (II) suivante : 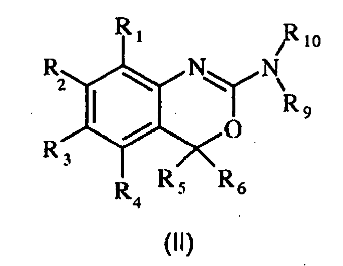 dans laquelle :
- R₁, R₂, R₃, et R₄, identiques ou différents, représentent un atome d'hydrogène, un atome d'halogène, notamment choisi parmi F, CI, Br, ou I, un groupe hydroxy, ou un groupe alkoxy de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxy, alkoxy de 1 ou 2 atomes de carbone, trifluorométhyle ou nitro ;
- R₉ et R₁₀, identiques ou différents, représentent un atome d'hydrogène, un groupe hydroxy, ou un groupe alkyle ou hydroxyalkyle de 1 à 3 atomes de carbone;
ou un sel pharmaceutiquement acceptable de celui-ci,
pour la préparation d'un médicament destiné à la prévention ou au traitement des troubles mnésiques.

La présente invention concerne également un composé de formule (II) telle que définie ci-dessus, ou un sel pharmaceutiquement acceptable de celui-ci, comme médicament pour la prévention ou le traitement des troubles mnésiques.

Dans un mode de réalisation particulier de l'invention, le composé de formule (II) est associé à au moins un composé additionnel destiné à la prévention
ou au traitement d'une pathologie liée à ou à l'origine d'un trouble mnésique.

La présente invention concerne également une composition pharmaceutique comprenant à titre de substance active :
- au moins un composé de formule (II) telle que définie ci-dessus, ou un sel pharmaceutiquement acceptable de celui-ci, et
- au moins un composé additionnel destiné à la prévention ou au traitement d'une pathologie liée à ou à l'origine d'un trouble mnésique,
   en association avec un véhicule pharmaceutiquement acceptable.

La présente invention concerne également des produits contenant :
- au moins un composé de formule (II) telle que définie ci-dessus, ou un sel pharmaceutiquement acceptable de celui-ci, et
- au moins un composé additionnel destiné à la prévention ou au traitement d'une pathologie liée à ou à l'origine d'un trouble mnésique,
   comme produit de combinaison pour une utilisation séparée, simultanée ou étalée dans le temps pour la prévention ou le traitement des troubles mnésiques.

### Description détaillée de l'invention

### Composés de formules (II)

La synthèse des composés de formule (II) définis ci-dessus peut être aisément mise en oeuvre à partir des enseignements du brevet français n °1 571 287.

Les sels pharmaceutiquement acceptables selon l'invention apparaîtront de manière évidente pour l'homme du métier, en particulier les sels de chlorhydrate des composés de formule (II) selon l'invention sont préférés.

Font également partie de l'invention les formes optiquement actives du composé de formule (II), tels que les énantiomères suivants (lorsque R₅ et R₆ sont différents) : 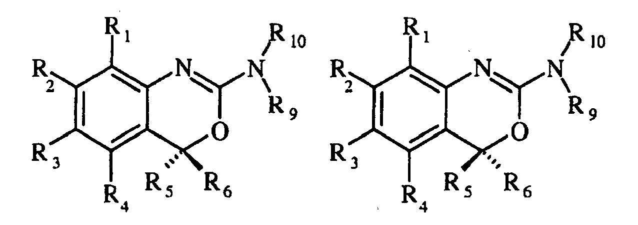 ou leurs mélanges, en particulier leur mélange racémique.

Le composé de formule (II) selon l'invention, est représenté, de manière particulièrement préférée, par le composé de formule (III) ou (IV) suivante :

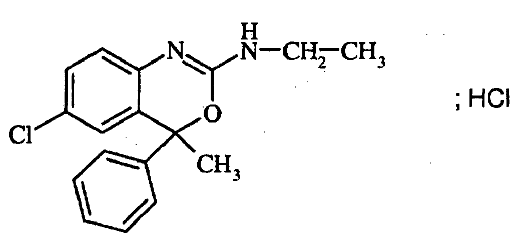

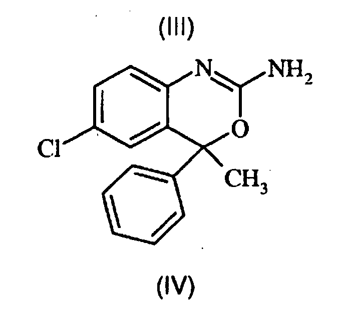

Le composé de formule (III) est l'étifoxine, ou chlorhydrate de 6-chloro-2-éthylamino-4-méthyl-4-phényl-4H-[3,1]benzoxazine.

Le composé de formule (IV), la déséthyl-étifoxine ou 2-amino-6-chloro-4-méthyl-4-phényl-4H-[3,1]benzoxazine, est un métabolite de l'étifoxine.

Font également partie de l'invention, les formes optiquement actives du composé de formule (III) selon l'invention, tels que les énantiomères suivants : 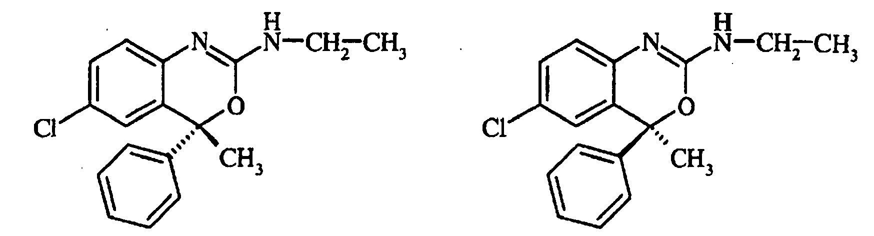 ou leurs mélanges, en particulier leur mélange racémique, notamment sous forme chlorhydrate, ainsi que les formes optiquement actives du composé de formule (IV) selon l'invention, tels que les énantiomères suivants : 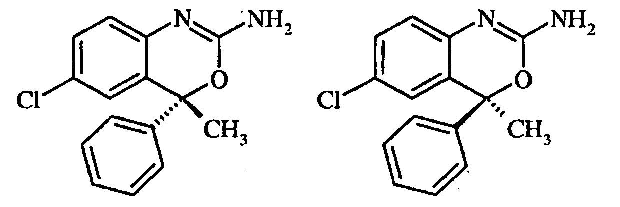 ou leurs mélanges, en particulier leur mélange racémique.

### Troubles mnésiques

Un individu est dit atteint d'un « trouble mnésique », s'il présente un déficit dans la capacité d'apprentissage de nouvelles informations ou une incapacité à se souvenir d'informations apprises ou d'évènements s'étant passés antérieurement à l'apparition du trouble.

Les troubles mnésiques ou troubles de la mémoire sont notamment définis dans les pages 123 à 163 du manuel de référence « Diagnostic and Statistical Manual IV » (DSM IV) dans le cadre des pathologies constituées du delirium, des démences et des troubles amnésiques.

Les troubles amnésiques, qui sont donc inclus dans les troubles mnésiques, sont notamment caractérisés par une altération de la mémoire en l'absence d'autres altérations cognitives significatives. Leur classement repose sur leur étiologie présumée, ces troubles pouvant être dus à une affection médicale générale, être induits par une substance, ou être non spécifiés.

Ainsi, comme on l'entend ici, les troubles mnésiques comprennent l'ensemble des formes d'amnésie, notamment l'amnésie antérograde, l'amnésie rétrograde, l'amnésie globale transitoire, l'ictus amnésique ou encore le syndrome amnésique pur.

Les troubles mnésiques selon l'invention peuvent notamment être dus à l'action de substances exogènes (c'est-à-dire que le trouble mnésique est d'origine iatrogène) ou être dus à des lésions organiques. Ces lésions peuvent notamment être de type dégénérative, ischémique, hémorragique, traumatique ou dues à des carences, par exemple en vitamines. L'étiologie non démentielle des troubles mnésiques est notamment résumée par Michel & Sellal (2006) Le Concours Médical 128:487-491.

Ainsi, de manière préférentielle, les troubles mnésiques sont liés aux ou provoqués par les pathologies, choisies dans la liste constituée de :
- un accident traumatique cérébral, tel qu'un traumatisme crânien ;
- une infection cérébrale, telle qu'une méningite ou une encéphalite, notamment limbique, herpétique, ou paranéoplasique ;
- une maladie neurodégénérative et/ou une démence, telle que la maladie d'Alzheimer, notamment sous sa forme prodomale ou à expression frontale, la maladie de Pick, la démence sémantique, la démence fronto-temporale, la démence cortico-basale, la démence à corps de Lewy diffus, la Chorée de Huntington, la maladie de Creutzfeldt-Jakob, l'atrophie corticale postérieure, ou l'atrophie multisystématisée (MSA) ;
- une affection démyélinisante, telle que la sclérose en plaque ;
- un accident vasculaire cérébral, ischémique ou hémorragique, tel qu'un infarctus hippocampique, un hématome sous-dural chronique, une anoxie cérébrale,
- une crise épileptique ;
- une tumeur cérébrale ;
- un état carentiel, tel que l'encéphalopathie de Gayet-Wernicke ou le syndrome de Korsakoff ;
- un trouble mnésique iatrogène, notamment dû à l'administration de composés pharmacologiques, tels que les sédatifs, les benzodiazépines et substances apparentées, les antinauséeux et antivertigineux, les antidépresseurs à action anticholinergique, les antihypertenseurs à action centrale, les corticostéroïdes, ou les bétabloquants franchissant la barrière hématoencéphalique
- une intoxication, notamment à l'alcool, l'oxyde de carbone, au mercure, au bismuth, au lithium, ou au plomb (saturnisme) ;
- une insuffisance d'organe, telle qu'une insuffisance hépatique chronique
ou une insuffisance thyroïdienne ;
- une paralysie, telle qu'une paralysie générale, une paralysie supranucléaire, une apraxie progressive primaire, une anarthrie progressive frontale, ou une aphasie progressive ;
- un trouble du comportement, tel qu'un trouble anxieux, un trouble dépressif primaire, ou un état de stress ;
- un désordre hydro-électrolytique ou métabolique.

Plus préférentiellement, les troubles mnésiques sont des troubles mnésiques iatrogènes, notamment dus à l'administration de composés pharmacologiques, tels que les sédatifs, les benzodiazépines et substances apparentées, les antinauséeux et antivertigineux, les antidépresseurs à action anticholinergique, les antihypertenseurs à action centrale, les corticostéroïdes, ou les bétabloquants franchissant la barrière hématoencéphalique.

### Composé additionnel

Comme il apparaîtra clairement à l'homme du métier, le composé additionnel n'est pas un composé de formule (II) selon l'invention.

De préférence, le composé additionnel est destiné au traitement d'une pathologie liée à ou provoquée par un trouble mnésique choisi dans la liste constituée de :
- un accident traumatique cérébral, tel qu'un traumatisme crânien ;
- une infection cérébrale, telle qu'une méningite ou une encéphalite, notamment limbique, herpétique, ou paranéoplasique ;
- une maladie neurodégénérative et/ou une démence, telle que la maladie d'Alzheimer, notamment sous sa forme prodomale ou à expression frontale, la maladie de Pick, la démence sémantique, la démence fronto-temporale, la démence cortico-basale, la démence à corps de Lewy diffus, la Chorée de Huntington, la maladie de Creutzfeldt-Jakob, l'atrophie corticale postérieure, ou l'atrophie multisystématisée (MSA) ;
- une affection démyélinisante, telle que la sclérose en plaque ;
- un accident vasculaire cérébral, ischémique ou hémorragique, tel qu'un infarctus hippocampique, un hématome sous-dural chronique, une anoxie cérébrale,
- une crise épileptique ;
- une tumeur cérébrale ;
- un état carentiel, tel que l'encéphalopathie de Gayet-Wernicke ou le syndrome de Korsakoff ;
- un trouble mnésique iatrogène, notamment dû à l'administration de composés pharmacologiques, tels que les sédatifs, les benzodiazépines et substances apparentées, les antinauséeux et antivertigineux, les antidépresseurs à action anticholinergique, les antihypertenseurs à action centrale, les corticostéroïdes, ou les bétabloquants franchissant la barrière hématoencéphalique
- une intoxication, notamment à l'alcool, l'oxyde de carbone, au mercure, au bismuth, au lithium, ou au plomb (saturnisme) ;
- une insuffisance d'organe, telle qu'une insuffisance hépatique chronique
ou une insuffisance thyroïdienne ;
- une paralysie, telle qu'une paralysie générale, une paralysie supranucléaire, une apraxie progressive primaire, une anarthrie progressive frontale, ou une aphasie progressive ;
- un trouble du comportement, tel qu'un trouble anxieux, un trouble dépressif primaire, ou un état de stress ;
- un désordre hydro-électrolytique ou métabolique.

Par ailleurs, le composé additionnel défini ci-dessus est de préférence choisi dans la liste comprenant ou constituée de :
- un composé destiné au traitement de la maladie d'Alzheimer, notamment :
   - un anticholinestérasique, tel que :
      ➢ le donépézil,
      ➢ la galantamine,
      ➢ la rivastigmine,
   - un antagoniste des récepteurs NMDA, tel que :
      ➢ la mémantine,
- un composé destiné au traitement des AVC (accidents vasculaires cérébraux) ischémiques en phase aiguë (altéphase) et de leurs suites (dihydroergocristine, piracétam), notamment selon le type d'AVC :
   ➢ l'héparine,
   ➢ l'aspirine,
   ➢ la nicardipine,

- un composé destiné au traitement des hémorragies cérébrales, notamment :
   ➢ la nimodipine,

- un antidépresseur, notamment :
   - un imipraminique, tel que :

➢ l'imipramine
➢ la clomipramine
➢ l'amoxapine,
➢ l'amitriptyline,
   - un inhibiteur sélectif de la recapture de la sérotonine, tel que :
      ➢ la fluoxétine,
      ➢ la paroxétine,
      ➢ le citalopram,
      ➢ la fluvoxamine,
      • un inhibiteur sélectif de la recapture de la sérotonine et de la noradrénaline, tel que :

      ➢ la venlafaxine,
      ➢ la mirtazapine,
      ➢ la tianeptine,

- un composé destiné au traitement des tumeurs cérébrales, notamment un agent de chimiothérapie,
- un composé destiné au traitement de la polynévrite alcoolique, notamment la vitamine B1.

### Administration

De préférence, le médicament, la composition pharmaceutique ou le produit selon l'invention comprennent une dose unitaire d'environ 25 mg à environ 2500 mg, notamment d'environ 100 à 1000 mg du composé de formule (II) telle que définie ci-dessus, ou du sel pharmaceutiquement acceptable de celui-ci.

De préférence également, le composé de formule (II) telle que définie ci-dessus, ou le sel pharmaceutiquement acceptable de celui-ci est administrée à une dose d'environ 25 mg/j à environ 2500 mg/j, notamment d'environ 100 mg/j à environ 1000 mg/j.

De préférence, le médicament, la composition pharmaceutique ou le produit selon l'invention convient pour une administration par voie orale.

De préférence également, le médicament, la composition pharmaceutique ou le produit selon l'invention se présente sous la forme d'une poudre, de cachets, de gélules ou de sachets.

### DESCRIPTION DES FIGURES

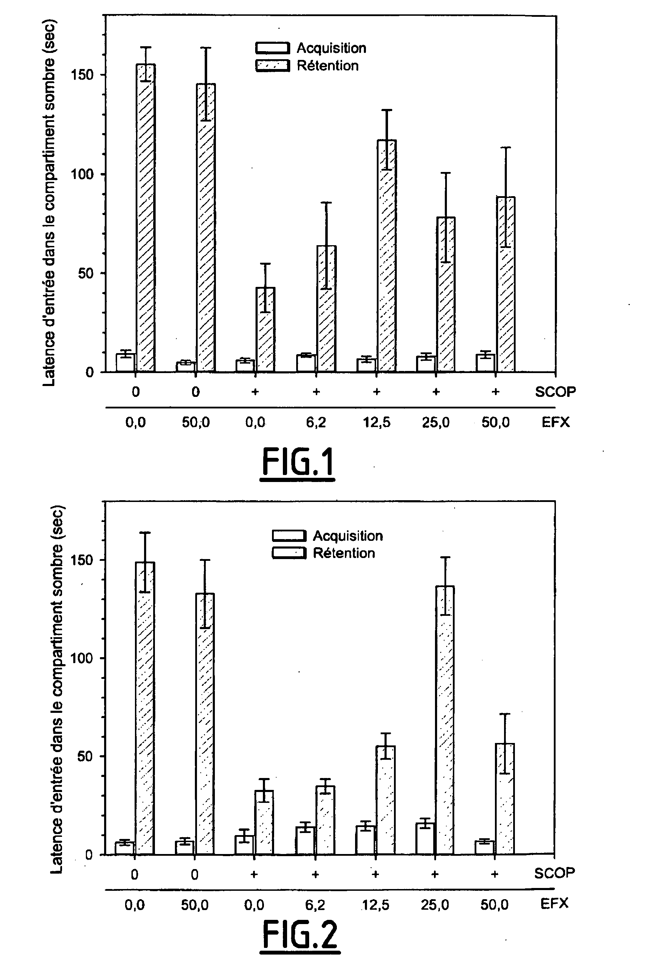

Figure 1 : Histogrammes représentant la moyenne du temps de latence d'entrée dans le compartiment sombre au moment de l'acquisition de l'information (barres blanches) ou au moment de la rétention de l'information (barres hachurées) en fonction des différents traitements réalisés. La scopolamine (SCOP) ou son véhicule (dose 0) ont été administrés 30 minutes avant l'acquisition à la dose de 1,4 mg/kg. L'étifoxine (EFX-mg/kg) a été administrée 35 minutes avant l'acquisition aux doses indiquées.

Figure 2 : Histogrammes représentant la moyenne du temps de latence d'entrée dans le compartiment sombre au moment de l'acquisition de l'information (barres blanches) ou au moment de la rétention de l'information (barres hachurées) en fonction des différents traitements réalisés. La scopolamine (SCOP) ou son véhicule (dose 0) ont été administrés 30 minutes avant l'acquisition à la dose de 1,4 mg/kg. L'étifoxine (EFX-mg/kg) a été administrée immédiatement après l'acquisition aux doses indiquées.

### EXEMPLE

### Etude des effets de l'étifoxine dans un modèle d'amnésie induite par la scopolamine chez le rat.

### -Objectif

Le but de l'étude a consisté à étudier les effets de l'étifoxine vis-à-vis de l'amnésie induite par une substance antagoniste des récepteurs cholinergiques chez le rat, la scopolamine (voir notamment Rush (1988) Behav. Neural. Biol. 1988 50:255-274).

### -Protocole

Les capacités de mémorisation sont évaluées au cours de l'épreuve de l'évitement passif dans une boîte à deux compartiments sombre et clair. Dans une première étape, dite d'acquisition, l'entrée dans le compartiment sombre est associée à un choc électrique désagréable. Dans une deuxième étape, dite de rétention, on mesure la latence d'entrée dans le compartiment sombre. Plus cette latence est faible, plus l'animal souffre d'amnésie.

L'amnésie est induite par l'administration de scopolamine 30 minutes avant l'étape d'acquisition de l'information.

Deux séries de test ont été réalisées :
1) L'étifoxine a été administrée par voie intrapéritonéale 35 minutes avant l'acquisition de l'événement.
   7 groupes de 10 à 20 rats ont été formés, chaque groupe recevant les traitements respectifs suivants :
   - Groupe 1 (témoin) : scopolamine 0 mg/kg, étifoxine 0 mg/kg
   - Groupe 2 (contrôle) : scopolamine 0 mg/kg, étifoxine 50 mg/kg
   - Groupe 3 : scopolamine 1,4 mg/kg, étifoxine 0 mg/kg
   - Groupe 4 : scopolamine 1,4 mg/kg, étifoxine 6,2 mg/kg
   - Groupe 5 : scopolamine 1,4 mg/kg, étifoxine 12,5 mg/kg
   - Groupe 6 : scopolamine 1,4 mg/kg, étifoxine 25 mg/kg
   - Groupe 7 : scopolamine 1,4 mg/kg, étifoxine 50 mg/kg
2) L'étifoxine a été administrée par voie intrapéritonéale immédiatement après l'acquisition de l'événement.
   7 groupes de 10 rats ont été formés, chaque groupe recevant les traitements respectifs suivants :
   - Groupe 1 (témoin) : scopolamine 0 mg/kg, étifoxine 0 mg/kg
   - Groupe 2 (contrôle) : scopolamine 0 mg/kg, étifoxine 50 mg/kg
   - Groupe 3 : scopolamine 1,4 mg/kg, étifoxine 0 mg/kg
   - Groupe 4 : scopolamine 1,4 mg/kg, étifoxine 6,2 mg/kg
   - Groupe 5 : scopolamine 1,4 mg/kg, étifoxine 12,5 mg/kg
   - Groupe 6 : scopolamine 1,4 mg/kg, étifoxine 25 mg/kg
   - Groupe 7 : scopolamine 1,4 mg/kg, étifoxine 50 mg/kg

Pour chaque série de test, et pour chaque groupe, la latence d'entrée dans le compartiment sombre a été mesurée lors de l'acquisition et lors de la rétention.

Pour la première série de tests, une analyse ANOVA à un facteur suivie du test de Student-Newman-Keuls a été réalisée afin de localiser les différences entre les groupes. La différence est considérée comme significative lorsque la valeur critique P obtenue était inférieure à 0,05.

Pour la deuxième série de tests, l'épreuve non paramétrique de Kruskal-Wallis suivie du test de Dunn a été réalisée afin de localiser les différences entre les groupes. La différence est considérée comme significative lorsque la valeur critique P obtenue était inférieure à 0,05.

### -Résultats

La scopolamine, administrée 30 minutes avant l'acquisition, provoque une amnésie chez le rat comme le traduit le court délai pour entrer dans le compartiment sombre, significativement plus faible que le délai observé chez les groupes témoins (Fig. 1,2 ; (SCOP +, EFX 0) vs (SCOP 0, EFX 0)).

Dans les deux schémas d'administration, on observe que l'étifoxine s'oppose aux effets amnésiants de la scopolamine selon une courbe en U inversée (Fig. 1, Fig. 2).

## Revendications

1. Utilisation d'au moins un composé de formule (II) suivante : 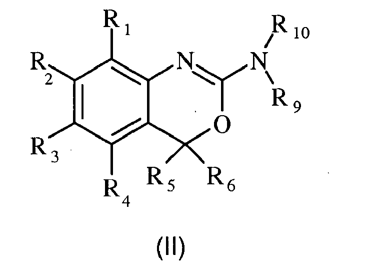 dans laquelle :
- R₁, R₂, R₃, et R₄, identiques ou différents, représentent un atome d'hydrogène, un atome d'halogène, notamment choisi parmi F, Cl, Br, ou I, un groupe hydroxy, ou un groupe alkoxy de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxy, alkoxy de 1 ou 2 atomes de carbone, trifluorométhyle ou nitro ;
- R₉ et R₁₀, identiques ou différents, représentant un atome d'hydrogène, un groupe hydroxy, ou un groupe alkyle ou hydroxyalkyle de 1 à 3 atomes de carbone ;
ou d'au moins un sel pharmaceutiquement acceptable de celui-ci,
pour la préparation d'un médicament destiné à la prévention ou au traitement des troubles mnésiques, en particulier des troubles amnésiques.

2. Utilisation selon la revendication 1, des composés de formules (III) et (IV) suivantes :

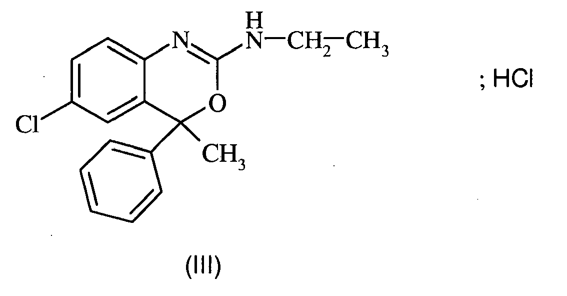

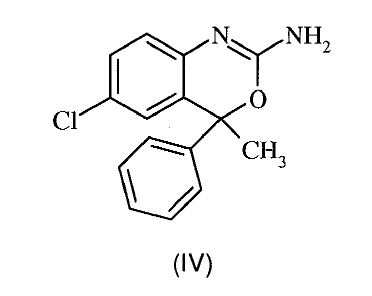

3. Utilisation selon la revendication 1 ou 2, dans laquelle le médicament convient pour l'administration d'une dose unitaire d'environ 25 mg à environ 2500 mg, notamment d'environ 100 à 1000 mg, du composé tel que défini dans l'une des revendications 1 à 2, ou d'un sel pharmaceutiquement acceptable de celui-ci.

4. Utilisation selon l'une des revendications 1 à 3, dans laquelle le médicament convient pour une administration par voie orale.

5. Utilisation selon l'une des revendications 1 à 4, dans laquelle le médicament se présente sous la forme d'une poudre, de cachets, de gélules ou de sachets.

6. Utilisation selon l'une des revendications 1 à 5, dans laquelle le composé tel que défini dans l'une des revendications 1 à 2 est associé à au moins un composé additionnel destiné à la prévention ou au traitement d'une pathologie liée à un trouble mnésique.

7. Utilisation selon la revendication 6, dans laquelle le composé additionnel destiné à la prévention ou au traitement d'une pathologie liée à un trouble mnésique est sélectionné dans le groupe constitué d'un anticholestérasique, tel que le donépézil ou la galantamine, de la dihydroergocristine, du piracétam et de la vitamine B1.

8. Composition pharmaceutique comprenant à titre de substance active :
- au moins un composé de formule (II) tel que défini dans l'une des revendications 1 à 2, ou un sel pharmaceutiquement acceptable de celui-ci, et
- au moins un composé additionnel destiné à la prévention ou au traitement d'une pathologie liée à un trouble mnésique,
en association avec un véhicule pharmaceutiquement acceptable,
pour son utilisation dans la prévention ou le traitement des troubles mnésiques, en particulier des troubles amnésiques.

9. Composition pharmaceutique pour son utilisation selon la revendication 8, dans laquelle le composé additionnel destiné à la prévention ou au traitement d'une pathologie liée à un trouble mnésique est sélectionné dans le groupe constitué d'un anticholestérasique, tel que le donépézil ou la galantamine, de la dihydroergocristine, du piracétam et de la vitamine B1.

10. Produits contenant :
• au moins un composé de formule (II) tel que défini dans l'une des revendications 1 à 2, ou un sel pharmaceutiquement acceptable de celui-ci, et
• au moins un composé additionnel destiné à la prévention ou au traitement d'une pathologie liée à un trouble mnésique,
comme produit de combinaison pour une utilisation séparée, simultanée ou étalée dans le temps pour la prévention ou le traitement des troubles mnésiques, en particulier des troubles amnésiques.

11. Produits pour leur utilisation selon la revendication 10, dans lesquels le composé additionnel destiné à la prévention ou au traitement d'une pathologie liée à un trouble mnésique est sélectionné dans le groupe constitué d'un anticholestérasique, tel que le donépézil ou la galantamine, de la dihydroergocristine, du piracétam et de la vitamine B1.

12. Composé de formule (II) tel que défini dans l'une des revendications 1 à 2, ou un sel pharmaceutiquement acceptable de celui-ci, pour son utilisation comme médicament dans la prévention ou le traitement d'un trouble mnésique, en particulier d'un trouble amnésique.

13. Composé de formule (II), ou un sel pharmaceutiquement acceptable de celui-ci, pour son utilisation selon la revendication 12, associé à au moins un composé additionnel destiné à la prévention ou au traitement d'une pathologie liée à un trouble mnésique.

14. Composé de formule (II), ou un sel pharmaceutiquement acceptable de celui-ci, pour son utilisation selon la revendication 13, dans lesquels le composé additionnel destiné à la prévention ou au traitement d'une pathologie liée à un trouble mnésique est sélectionné dans le groupe constitué d'un anticholestérasique, tel que le donépézil ou la galantamine, de la dihydroergocristine, du piracétam et de la vitamine B1.

## Patentansprüche

1. Anwendung mindestens einer Verbindung der folgenden Formel (II) 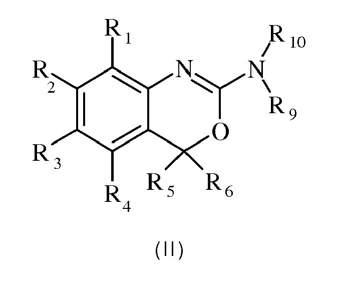 in der
- R₁, R₂, R₃ und R₄, gleich oder unterschiedlich, für ein Wasserstoffatom, ein Halogenatom, insbesondere unter F, Cl, Br und I ausgewählt, eine Hydroxy- oder eine Alkoxygruppe mit 1 oder 2 Kohlenstoffatomen stehen,
- R₅ und R₆, gleich oder unterschiedlich, für ein Wasserstoffatom, eine Alkyl- oder Cycloalkylgruppe mit 1 bis 6 Kohlenstoffatomen oder eine Arylgruppe mit 6 Kohlenstoffatomen, deren aromatischer Kern eventuell mit einem oder mehreren Halogenatomen oder einer oder mehreren Hydroxygruppen, Alkoxygruppen mit 1 oder 2 Kohlenstoffatomen, Trifluormethyl- oder Nitrogruppen substituiert ist,
- R₉ und R₁₀, gleich oder unterschiedlich, für ein Wasserstoffatom, eine Hydroxygruppe oder eine Alkyl- oder Hydroxyalkylgruppe mit 1 bis 3 Kohlenstoffatomen,
oder mindestens eines pharmazeutisch akzeptablen Salzes davon für die Zubereitung eines Arzneimittels zur Vorbeugung oder Behandlung mnestischer Störungen, insbesondere amnestischer Störungen.

2. Anwendung nach Patentanspruch 1 der Verbindungen mit den folgenden Formeln (III) und (IV)

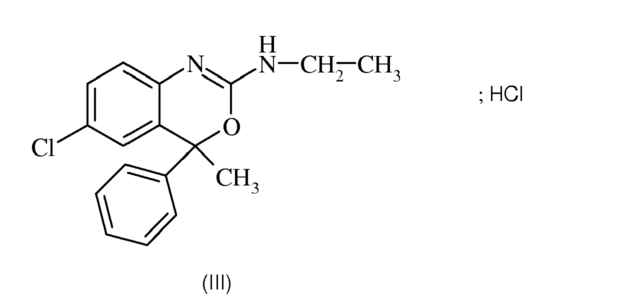

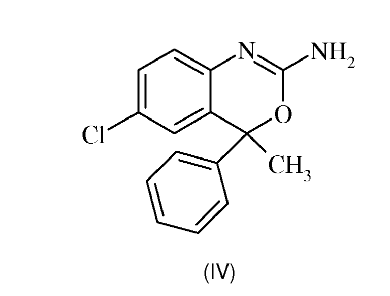

3. Anwendung nach Patentanspruch 1 oder 2, in der das Arzneimittel zur Verabreichung einer Einheitsdosis von ungefähr 25 mg bis ungefähr 2500 mg, insbesondere von ungefähr 100 bis 1000 mg der In einem der Patentansprüche 1 bis 2 definierten Verbindung oder eines pharmazeutisch akzeptablen Salzes davon geeignet ist.

4. Anwendung nach Patentanspruch 1 bis 3, in der das Arzneimittel zur oralen Verabreichung geeignet ist.

5. Anwendung nach einem der Patentansprüche 1 bis 4, in der das Arzneimittel die Form eines Pulvers, von Tabletten, Kapseln oder Beuteln hat.

6. Anwendung nach einem der Patentansprüche 1 bis 5, in der die In einem der Patentansprüche 1 bis 2 definierte Verbindung mit mindestens einer zusätzlichen Verbindung kombiniert wird, die zur Vorbeugung oder Behandlung einer Pathologie bestimmt ist, die mit einer mnestischen Störung verbunden ist.

7. Anwendung nach Patentanspruch 6, in der der die zur Vorbeugung oder Behandlung einer Pathologie, die mit einer mnestischen Störung verbunden ist, bestimmte zusätzliche Verbindung aus der Gruppe gewählt wurde, bestehend aus Cholesterinesterasehemmern wie etwa Donepezil oder Galantamin, Dihydroergocristin, Piracetam und Vitamin B1.

8. Pharmazeutische Zubereitung, als Wirkstoff enthaltend
- mindestens eine Verbindung der Formel (II), wie in einem der Patentansprüche 1 bis 2 definiert, oder eines pharmazeutisch akzeptablen Salzes davon und
- mindestens eine zusätzliche Verbindung, die zur Vorbeugung oder Behandlung einer Pathologie bestimmt ist, die mit einer mnestischen Störung verbunden ist,
in Kombination mit einem pharmazeutisch akzeptablen Vehikel, zur Anwendung in der Vorbeugung gegen oder Behandlung von mnestischen Störungen, insbesondere amnestischer Störungen.

9. Pharmazeutische Zubereitung zur Anwendung nach Patentanspruch 8, in der die zusätzliche Verbindung, die zur Vorbeugung oder Behandlung einer Pathologie bestimmt ist, die mit einer mnestischen Störung verbunden ist, aus der Gruppe gewählt wurde, bestehend aus Cholesterinesterasehemmern wie etwa Donepezil oder Galantamin, Dihydroergocristin, Piracetam und Vitamin B1.

10. Produkte, enthaltend
• mindestens eine Verbindung der Formel (II), wie in einem der Patentansprüche 1 bis 2 definiert, oder ein pharmazeutisch akzeptables Salz davon und
• mindestens eine zusätzliche Verbindung, die zur Vorbeugung oder Behandlung einer Pathologie bestimmt ist, die mit einer mnestischen Störung verbunden ist,
als Kombinationsprodukt für eine getrennte, gleichzeitige oder zeitlich gestaffelte Anwendung zur Vorbeugung oder Behandlung mnestischer Störungen, insbesondere amnestischer Störungen.

11. Produkte zur Anwendung nach Patentanspruch 10, in denen die zusätzliche Verbindung, die zur Vorbeugung oder Behandlung einer Pathologie bestimmt ist, die mit einer mnestischen Störung verbunden ist, aus der Gruppe gewählt wurde, bestehend aus Cholesterinesterasehemmern wie etwa Donepezil oder Galantamin, Dihydroergocristin, Piracetam und Vitamin B1.

12. Verbindung der Formel (II), wie in einem der Patentansprüche 1 bis 2 definiert, oder ein pharmazeutisch akzeptables Salz davon für die Anwendung als Arzneimittel in der Vorbeugung oder Behandlung einer mnestischen Störung, insbesondere einer amnestischen Störung.

13. Verbindung der Formel (II) oder ein pharmazeutisch akzeptables Salz davon zur Anwendung nach Patentanspruch 12, kombiniert mit mindestens einer zusätzlichen Verbindung, die zur Vorbeugung oder Behandlung einer Pathologie bestimmt ist, die mit einer mnestischen Störung verbunden ist.

14. Verbindung der Formel (II) oder ein pharmazeutisch akzeptables Salz davon zur Anwendung nach Patentanspruch 13, in der die zusätzliche Verbindung, die zur Vorbeugung oder Behandlung einer Pathologie bestimmt ist, die mit einer mnestischen Störung verbunden ist, aus der Gruppe gewählt wurde, bestehend aus Cholesterinesterasehemmern wie etwa Donepezil oder Galantamin, Dihydroergocristin, Piracetam und Vitamin B1.

## Claims

1. Use of at least one compound of the following formula (II): 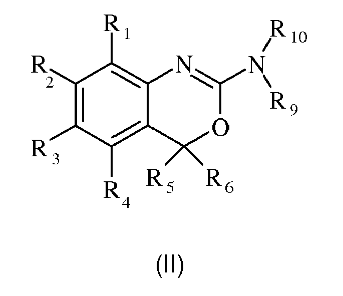 wherein:
• R₁, R₂, R₃, and R₄ are the same or different and represent a hydrogen atom, a halogen atom, selected in particular from F, Cl, Br or I, a hydroxy group, or an alkoxy group with 1 or 2 carbon atoms;
• R₅ and R₆ are the same or different and represent a hydrogen atom, an alkyl or cycloalkyl group with 1 to 6 carbon atoms, or an aryl group with 6 carbon atoms of which the aromatic ring is optionally substituted by one or more halogen atoms or one or more hydroxy groups, alkoxy groups with 1 or 2 carbon atoms, trifluoromethyl groups or nitro groups;
• R₉ and R₁₀ are the same or different and represent a hydrogen atom, a hydroxy group, or an alkyl or hydroxyalkyl group with 1 to 3 carbon atoms;
or of at least one pharmaceutically acceptable salt thereof,
for the manufacture of a medicament intended for the prevention or treatment of memory disorders, in particular of amnestic disorders.

2. The use according to claim 1, of compounds of the following formulae (III) and (IV):

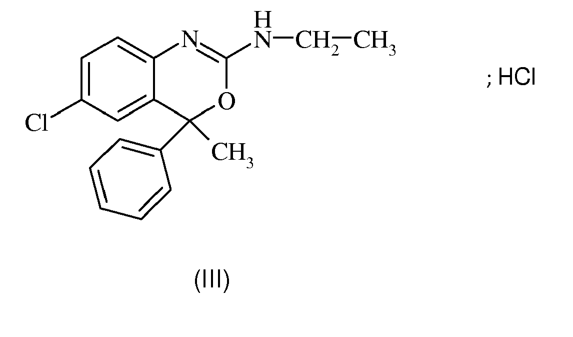

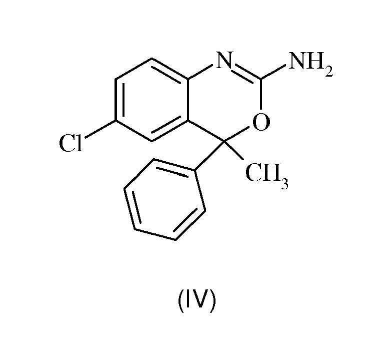

3. The use according to claim 1 or 2, wherein the medicament is suitable for the administration of a unit dose of approximately 25 mg to approximately 2500 mg, in particular of approximately 100 to 1000 mg, of the compound as defined in any of claims 1 to 2, or of a pharmaceutically acceptable salt thereof.

4. The use according to any of claims 1 to 3, wherein the medicament is suitable for an administration by the oral route.

5. The use according to any of claims 1 to 4, wherein the medicament is in the form of a powder, cachets, capsules or sachets.

6. The use according to any of claims 1 to 5, wherein the compound as defined in any of claims 1 to 2 is associated with at least one additional compound intended for the prevention or treatment of a pathology associated with a memory disorder.

7. The use according to claim 6, wherein the additional compound intended for the prevention or treatment of a pathology associated with a memory disorder is selected from the group consisting of an anticholinesterase, such as donepezil or galantamine, dihydroergocristine, piracetam, and vitamin B1.

8. A pharmaceutical composition comprising as an active substance:
- at least one compound of formula (II) as defined in any of claims 1 to 2, or a pharmaceutically acceptable salt thereof, and
- at least one additional compound intended for the prevention or treatment of a pathology associated with a memory disorder,
in association with a pharmaceutically acceptable excipient,
for use in the prevention or treatment of memory disorders, in particular of amnestic disorders.

9. The pharmaceutical composition for use according to claim 8, wherein the additional compound intended for the prevention or treatment of a pathology associated with a memory disorder is selected from the group consisting of an anticholinesterase, such as donepezil or galantamine, dihydroergocristine, piracetam, and vitamin B1.

10. Products containing:
• at least one compound of formula (II) as defined in any of claims 1 to 2, or a pharmaceutically acceptable salt thereof, and
• at least one additional compound intended for the prevention or treatment of a pathology associated with a memory disorder,
as a combined preparation for separate, simultaneous or sequential use in the prevention or treatment of memory disorders, in particular amnestic disorders.

11. Products for use according to claim 10, wherein the additional compound intended for the prevention or treatment of a pathology associated with a memory disorder is selected from the group consisting of an anticholinesterase, such as donepezil or galantamine, dihydroergocristine, piracetam, and vitamin B1.

12. A compound of formula (II) as defined in any of claims 1 to 2, or a pharmaceutical salt thereof, for use as a medicament in the prevention or treatment of a memory disorder, in particular an amnestic disorder.

13. The compound of formula (II), or a pharmaceutical salt thereof, for use according to claim 12, associated with at least one additional compound intended for the prevention or treatment of a pathology associated with a memory disorder.

14. The compound of formula (II), or a pharmaceutical salt thereof, for use according to claim 13, wherein the additional compound intended for the prevention or treatment of a pathology associated with a memory disorder is selected from the group consisting of an anticholinesterase, such as donepezil or galantamine, dihydroergocristine, piracetam, and vitamin B1.
